# EUROPÄISCHE PATENTSCHRIFT

(11) **EP 0 648 725 B1**
(45) Veröffentlichungstag und Bekanntmachung des Hinweises auf die Patenterteilung: **15.04.1998**
(21) Anmeldenummer: 94115810.7
(22) Anmeldetag: 07.10.1994
(51) Int. Cl.: C07C 17/358, C07C 19/12, B01J 27/12, B01J 27/125, B01J 27/128

(54) **Chlorfluorkohlen(wasser)stoff-Umwandlungsverfahren**
Process for isomerizing chlorofluorohydrocarbons
Procédé pour l'isomérisation d'hydrocarbures chlorofluorés

(30) Priorität: 15.10.1993 DE 4335178; 11.12.1993 DE 4342330
(43) Veröffentlichungstag der Anmeldung: 19.04.1995
(73) Patentinhaber: Solvay Fluor und Derivate GmbH, D-30173 Hannover (DE)
(72) Erfinder: Swidersky, Hans-Walter, Dr., D-30175 Hannover (DE); Eicher, Johannes, Dr., D-74229 Oedheim (DE); Born, Thomas, D-31188 Holle (DE); Brosch, Carsten, D-30926 Seelze (DE); Rudolph, Werner, Dr., D-30559 Hannover (DE)
(74) Vertreter: Lauer, Dieter, Dr.

(56) Entgegenhaltungen:
- EP-A- 0 404 297
- DE-A- 1 668 346
- DE-A- 3 013 888

## Beschreibung

Die Erfindung betrifft ein Verfahren zur Herstellung von Verbindungen der Formel (I) CF₃CCl₂X mit X = Cl, H durch Umlagerung unter Verwendung eines AlCl₃-Katalysators.

CF₃CCl₃ ist ein Ausgangsprodukt für die chemische Synthese. Beispielsweise kann es mit SO₃ zu Trifluoracetylchlorid umgesetzt werden, welches seinerseits in der chemischen Synthese eingesetzt wird. Durch Hydrierung von CF₃CCl₃ (R113a) können Chlorfluorkohlenwasserstoffe hergestellt werden, welche als Ersatzstoffe für vollhalogenierte Kohlenstoffverbindungen als Lösungsmittel oder Treibmittel Anwendung finden. CF₃CHCl₂ ist ein solches Lösungsmittel oder Treibmittel. Es ist bereits bekannt, daß man beispielsweise CF₃CCl₃ durch Umlagerung von CF₂ClCFCl₂ über Aluminiumchlorid herstellen kann. Nachteil dieser Reaktion ist es, daß die Umsetzung erst bei höherer Temperatur anspringt, dann aber mit hoher Wärmeentwicklung erfolgt, so daß oft ein Durchgehen des Ansatzes zu beobachten ist.

Die DE-A 1 668 346 offenbart ein Verfahren zur Herstellung von 1,1,1-Trifluortrichlorethan durch Umlagerung von 1,1,2-Trifluortrichlorethan über Aluminiumchlorid-Pulver als Katalysator. Vor der Anwendung wird das Aluminiumchlorid-Pulver bei einer Temperatur oberhalb von 30°C durch Kontaktieren mit 1,1,2-Trifluortrichlorethan aktiviert.

Die EP-A 404 297 offenbart ein Verfahren zur Isomerisierung von Chlorfluorethanen über Aluminiumtrihalogenid. Das Aluminiumtrihalogenid wird in situ bei der Reaktionsdurchführung aktiviert, indem man dem Reaktionsgefäß Chrom, Mangan, Molybdän, Wolfram oder Edelstahl zufügt. Man kann auch in einem Edelstahlreaktor arbeiten, statt Edelstahl zuzusetzen. Das Verfahren eignet sich beispielsweise zur Isomerisierung von 1,1,2-Trifluortrichlorethan.

Aufgabe der vorliegenden Erfindung ist es, ein Verfahren zur Herstellung von CF₃CCl₃ und CF₃CHCl₂ durch Umlagerung über Aluminiumchlorid anzugeben, welches mit kürzeren Reaktionszeiten, unter verringerter Bildung von Nebenprodukten und insbesondere verringerter Neigung zum Durchgehen der Reaktionen durchgeführt werden kann. Diese Aufgabe wird durch das Verfahren der vorliegenden Erfindung gelöst.

Das erfindungsgemäße Verfahren zur Herstellung von Verbindungen der Formel (I) CF₃CCl₂X mit X = Cl, H durch Umlagerung von CF₂ClCFCl₂, falls X = Cl ist, oder durch Umlagerung von CF₂ClCHClF oder CF₂HCCl₂F, falls X = H ist, über AlCl₃ als Katalysator, ist dadurch gekennzeichnet, daß man als Katalysator AlCl₃ einsetzt, welches mittels eines Metallhalogenids, ausgewählt aus der Gruppe umfassend AgCl, FeCl₃ und saure Salze der Formel (II) NaF·nHF mit 0 < n ≤ 2 aktiviert worden ist. Dabei erfolgt die Aktivierung mit möglichst wasserfreien Verbindungen.

Eine bevorzugte Ausführungsform sieht vor, daß man CF₃CCl₃ aus CF₂ClCFCl₂ herstellt. Anhand dieser bevorzugten Ausführungsform wird die Erfindung weiter erläutert.

Es ist möglich, reines CF₂ClCFCl₂ einzusetzen. Vorzugsweise setzt man dieses Ausgangsverbindung in Form eines Gemisches aus CF₂ClCFCl₂ und CF₃CCl₃ ein, wie es bei technischen Verfahren anfällt.

Für den Fall der Aktivierung mit einem Salz der Formel (II) ist der Parameter n vorzugsweise größer als 0,5 und kleiner oder gleich 2. Für den Fachmann ist klar, daß, falls Gemische mit n < 1 vorliegen, hier Gemische von NaF zusammen mit HF-Addukten von NaF vorliegen. Bevorzugt verwendet man Verbindungen der Formel (II), deren Zusammensetzung innerhalb des Bereiches liegt, dessen Grenzen durch die Verbindungen NaF·0,8 HF und NaF·1,5 HF dargestellt werden. Bevorzugte Verbindung der Formel (II) ist NaF·HF (d. h. n = 1).

Die Umlagerung wird bevorzugt bei einer Temperatur zwischen 70° und 95 °C durchgeführt. Man kann die Umlagerung auch bei niedrigeren Temperaturen durchführen; dann ist der Umwandlungsgrad möglicherweise geringer. Man kann die Umlagerung auch bei höheren Temperaturen durchführen; dabei kann es zur vermehrten Bildung von Nebenprodukten kommen. Wegen der einsetzenden exothermen Reaktion ist es empfehlenswert, das Reaktionsgemisch zunächst langsam auf Temperaturen im Bereich von 70° bis 80 °C zu erwärmen und, sofern die exotherme Reaktion einsetzt, die Heizleistung entsprechend einzuregeln bzw. sogar eine Kühlung vorzusehen, sofern man im besonders bevorzugten Bereich von 75° bis 90 °C arbeiten will.

Zweckmäßig beträgt der Gehalt an Metallhalogenid im aktivierten Katalysator mindestens 40 ppm an Fe, Ag oder Na in Form von FeCl₃, AgCl oder NaF · nHF. Besonders gute Ergebnisse bezüglich der Selektivität und der Reaktionsgeschwindigkeit bei der Umlagerung werden erzielt, wenn das aktivierende Metallhalogenid, z.B. saure Salz der Formel (II) und AlCl₃ im Gewichtsverhältnis zwischen 1:5 und 1:100, insbesondere zwischen 1:10 und 1:100 eingesetzt werden. Der aktivierte Katalysator liegt vorzugsweise in einer Menge von 0,1 bis 10 Gew.-% (Gesamtgewicht des aktivierenden Metallhalogenids, z.B. des Salzes der Formel (II) und des AlCl₃) im Reaktionsgemisch vor.

Die erfindungsgemäße Umlagerung kann man bei Normaldruck, gewünschtenfalls auch erniedrigtem oder erhöhtem Druck, z. B. bis hin zu 5 bar (abs.) durchführen.

Ein weiterer Gegenstand der Erfindung ist ein die Umlagerung von Chlorfluorkohlen(wasser)stoffen katalysierender Umlagerungskatalysator auf Basis von Aluminiumchlorid, der erhältlich ist durch Kontaktieren von AlCl₃ und einem Metallhalogenid, ausgewählt aus der Gruppe umfassend AgCl, FeCl₃ und saure Salze der Formel (II). Ein bevorzugter Umlagerungskatalysator ist erhältlich durch Kontaktieren von AlCl₃ und AgCl, FeCl₃ oder einem Salz der Formel NaF·nHF mit 0,5 < n ≤ 2, insbesondere in CF₂ClCCl₂F. Das "Kontaktieren" besteht vorzugsweise aus dem Vermischen von AlCl₃ und dem Metallhalogenid, vorzugsweise in CF₂ClCCl₂F. Dabei werden besonders wirksame Umlagerungskatalysatoren erhalten, wenn das Kontaktieren von AlCl₃ und des Metallhalogenids, insbesondere AgCl, FeCl₃ oder NaF·HF in CF₂ClCCl₂F über eine Zeitdauer von mindestens 1 Stunde durchgeführt wird, vorzugsweise über eine Zeitdauer zwischen 3 bis 10 Stunden. Das Gewichtsverhältnis zwischen AlCl₃ und dem Metallhalogenid liegt vorzugsweise im Bereich zwischen 5:1 und 100:1. Das Lösungsmittel kann dann gewünschtenfalls entfernt werden, z.B. durch Verdampfen oder Filtrieren.

Ein weiterer Gegenstand der Erfindung sind Aluminiumchlorid-Umlagerungskatalysatoren, gekennzeichnet durch einen Gehalt an einem Metallhalogenid, ausgewählt aus der Gruppe umfassend AgCl, FeCl₃ und sauren Salzen der Formel (II) mit der Maßgabe, daß Aluminiumchlorid mit einem Gehalt von bis zu 40 ppm Fe in Form von FeCl₃ ausgeschlossen ist. Handelsübliches Aluminiumtrichlorid p.A. enthält bis zu 40 ppm Fe in Form von FeCl₃ und wird nicht beansprucht. Bevorzugte Katalysatoren enthalten mehr als 40 bis hin zu 10.000 ppm, insbesondere 70 bis 150 ppm Fe, Ag bzw. Na in Form von FeCl₃, AgCl oder NaF·nHF.

Die Herstellung des letztgenannten Aluminiumchlorid-Umlagerungskatalysators gelingt beispielsweise durch Vermischen der angegebenen Bestandteile im gewünschten Gewichtsverhältnis bzw. unter Bildung des gewünschten Gewichtsverhältnisses oder der gewünschten Katalysator-Zusammensetzung, oder indem man von Silber- und/oder Eisen-haltigen Aluminiumverbindungen ausgeht und die Mischung in die entsprechenden Chloride überführt.

Die nach den vorstehend beschriebenen Verfahren erhaltenen Umlagerungskatalysatoren eignen sich für das erfindungsgemäße Umwandlungsverfahren. Dabei zeichnen sich die bevorzugten Umlagerungskatalysatoren durch besonders hohe katalytische Aktivität bei der Umlagerung aus.

Das erfindungsgemäße Verfahren wird zweckmäßig folgendermaßen durchgeführt: Zunächst erzeugt man eine, ebenfalls zur Erfindung gehörende Vormischung des Umlagerungskatalysators. Dabei vermischt man möglichst feinteiliges, beispielsweise pulverförmiges AlCl₃ mit der gewünschten Menge des ebenfalls feinteiligen Metallhalogenids. So kann man eine Vormischung erzeugen,welche aus 0,1 bis 200 Gewichtsteilen oder mehr an AlCl₃ möglichst feinteilig und 0,01 bis 1 Gewichtsteil des Metallhalogenids, z.B. des Salzes der Formel (II), insbesondere NaF·HF (ebenfalls möglichst feinteilig), sowie 0 bis 99,89 Gewichtsteilen CF₂ClCCl₂F besteht. Vorzugsweise besteht die Vormischung aus 0,1 bis 200 Gewichtsteilen pulverförmigem AlCl₃, 0,01 Gewichtsteilen des Metallhalogenids und 0 bis 99 Gewichtsteilen CF₂ClCCl₂F.

Eine andere Katalysatorvormischung zur Herstellung des Katalysators besteht aus 0,1 bis 20 Gewichtsteilen pulverförmigem AlCl₃, 0,01 Gewichtsteilen eines oder mehrer Metallhalogenide aus der Gruppe bestehend aus AgCl, FeCl₃ und sauren Salzen der Formel (II) mit O < n ≤ 2 und 0 bis 99 Gewichtsteilen CF₂ClCCl₂F.

Optimal erzeugt man eine Suspension der Feststoffe in CF₂ClCCl₂F mit einem Feststoffgehalt zwischen 0,1 und 10 Gew.-%. Diese Vormischung des Umlagerungskatalysators läßt man vorzugsweise mindestens 1 Stunde, insbesondere bis zu 10 Stunden, bei einer Temperatur von 10 bis 30 °C, vorzugsweise bei Umgebungstemperatur, stehen. Dabei soll die Mischung bevorzugt gerührt werden. Nach Ende der Kontaktzeit wird die Vormischung, die sich in den erfindungsgemäßen Umlagerungskatalysator umgewandelt hat, der umzulagernden Verbindung (bzw. Gemisch, das die umzulagernde Verbindung enthält) zugesetzt. Dann wird die Mischung aufgeheizt.

Alternativ geht man von Aluminiumchlorid aus, das herstellungsbedingt die gewünschte Menge an Silberchlorid oder Eisenchlorid enthält oder bei welchem man während der Herstellung den Aluminium-Ausgangsverbindungen Silber oder Silberverbindungen bzw. Eisenverbindungen oder Eisen zugesetzt hat.

Der Umwandlungsgrad bei der Verfahrensdurchführung kann durch übliche Untersuchungsmethoden, beispielsweise Gaschromatographie, verfolgt werden. Nach Beendigung der Umlagerung werden flüchtige Bestandteile abgedampft und in üblicher Weise, beispielsweise durch Destillation, abgetrennt. Der Umlagerungskatalysator kann wiederverwendet werden.

Das erfindungsgemäße Verfahren hat zunächst den Vorteil, daß kürzere Reaktionszeiten möglich sind. Beim herkömmlichen Verfahren ohne Aktivierung des Katalysators kann es zu deutlicher Verzögerung beim Start der Reaktion kommen, so daß die Reaktion oft durchgeht. Die Kontrolle der Reaktionsbedingungen ist beim erfindungsgemäßen Verfahren erleichtert. Zudem fallen weniger Nebenprodukte an.

Die Erfindung wird anhand der folgenden Beispiele weiter erläutert, ohne sie in ihrem Umfang einzuschränken.

### Beispiel 1:

### Herstellung der Vormischung des Umwandlungskatalysators und Herstellung des Umwandlungskatalysators

**1.1.** Ohne Ausdehnung der Kontaktzeit
   **1.1.1.** Gewichtsverhältnis von AlCl₃ zu NaF·HF 10:1
      30 g AlCl₃-Pulver (Produkt der Firma BASF) und 3 g NaF·HF (ebenfalls pulverförmig) wurden unter Zugabe von 300 g reinem R113 (CF₂ClCFCl₂) miteinander vermischt. Das Gemisch wurde sofort für die Umwandlung des zugegebenen R113 eingesetzt.
   **1.1.2.** Gewichtsverhältnis zwischen AlCl₃ und NaF·HF 6,7:1
      Beispiel 1.1.1. wurde wiederholt, diesmal wurden jedoch 20 g AlCl₃-Pulver mit 3 g NaF·HF unter Zusatz von 300 g eines Gemisches der Isomeren CF₂ClCFCl₂ und CF₃CCl₃ (R113/R113a) im Gewichtsverhältnis 10,3:89,7 vermischt. Auch hier erfolgte dann sofort die Anwendung für die Umwandlung.
**1.2.** Herstellung des Umwandlungskatalysators mit Ausdehnung der Kontaktzeit
   **1.2.1.** Kontaktzeit von 1,5 h
      30 g Aluminiumchloridpulver und 3 g NaF·HF wurden 1,5 Stunden lang in 300 g eines Isomerengemisches von R113 und R113a im Gewichtsverhältnis 12,6:87,4 kontaktiert.
   **1.2.2.** Ausdehnung der Kontaktzeit auf 5 Stunden
      30 g Aluminiumchloridpulver und 3 g NaF·HF wurden 5 Stunden lang in 300 g eines Isomerengemisches von R113 und R113a im Gewichtsverhältnis 10,3:89,7 bei Raumtemperatur kontaktiert.

### Beispiel 2:

### Durchführung der Umwandlungsreaktion

**2.1.** Durchführung unter Verwendung des gemäß Beispiel 1.1.1 hergestellten Katalysators
   Das gemäß Beispiel 1.1.1 hergestellte Gemisch aus Aluminiumchlorid, NaF·HF und R113 wurde erhitzt, bis die Reaktion ansprang (Temperatur von 79 °C). Das Gemisch wurde dann 20 Minuten lang bei einer Temperatur von maximal 108 °C gerührt. Flüchtige Bestandteile wurden abgedampft, das Verhältnis der Isomere 113 und 113a betrug 0,1:99,9. Die Summe der Nebenprodukte betrug 15,8 %.
**2.2.** Durchführung unter Verwendung des gemäß Beispiel 1.1.2. hergestellten Katalysators
   Das Gemisch aus Aluminiumchlorid, NaF·HF und dem Isomerengemisch R113/R113a wurde erhitzt, bis die Reaktion ansprang (etwa 77 °C). Das Gemisch wurde dann 30 Minuten lang bei einer Temperatur von maximal 81 °C gehalten. Das anfängliche Isomerenverhältnis R113/R113a von 10,3:89,7 hatte sich zu einem Verhältnis von 1,2:98,8 verschoben. Die Summe der Nebenprodukte betrug 7,9 %.
**2.3.** Durchführung mit einem Isomerengemisch R113/R113a im Verhältnis von 10,3:89,7
   30 g Aluminiumchloridpulver, 3 g NaF·HF und 300 g eines Isomerengemisches von R113 und R113a im Verhältnis 10,3:89,7 wurden ohne Ausdehnung der Aktivierungszeit, d. h. sofort nach dem Vermischen, erhitzt, bis die Reaktion ansprang (etwa 82 °C). Die Mischung wurde dann 10 Minuten lang bei einer Temperatur von maximal 88 °C gehalten. Die Endkonzentration des Isomerengemisches von R113/R113a betrug 0,03:99,97. Die Summe der Nebenprodukte lag bei 11,6 Gew.-%.
**2.4**. Durchführung unter Verwendung des gemäß 1.2.1. hergestellten Katalysators
   Das gemäß Beispiel 1.2.1. hergestellte Gemisch aus Aluminiumchlorid, NaF·HF und dem Isomerengemisch aus R113 und R113a wurde, nachdem es 1,5 Stunden lang bei Raumtemperatur gerührt worden war, erhitzt, bis die Reaktion ansprang (bei etwa 80 °C). Das Gemisch wurde dann 30 Minuten lang bei einer Temperatur von maximal 85 °C gehalten. Die Endkonzentration des Isomerengemisches R113/R113a betrug 0,03:99,97. Die Summe der Nebenprodukte betrug 13,8 Gew.-%.
**2.5.** Durchführung unter Verwendung des 5 Stunden lang aktivierten Katalysators aus Beispiel 1.2.2.
   Das Gemisch aus Aluminiumchlorid, NaF·HF und dem Isomerengemisch von R113/R113a, dessen Herstellung in Beispiel 1.2.2. beschrieben wurde, wurde 5 Stunden lang bei Raumtemperatur gerührt. Danach wurde das Gemisch bis zum Anspringen der Reaktion erhitzt (etwa 77 °C). Dann wurde es 30 Minuten lang bei einer Temperatur von maximal 79,5 °C gehalten. Das anfängliche Isomerenverhältnis von R113 zu R113a von 10,3:89,7 hatte sich zu einem Verhältnis von 0,05:99,95 verschoben. Die Summe der Nebenprodukte betrug 11,4 Gew.-%.

Wie die Beispiele zeigen, ist ein durch Verlängerung der Kontaktzeit erzeugter Umlagerungskatalysator besonders wirksam. Dies zeigt sich darin, daß bereits bei tieferer Reaktionstemperatur ein sehr hoher Umwandlungsgrad erzielt wird.

### Beispiel 3:

### Verwendung von Eisentrichlorid enthaltendem Aluminiumchlorid als Umlagerungskatalysator

Eingesetzt wurde Aluminiumchlorid, welches durch Zusatz von FeCl₃ 74 ppm Fe enthielt.

In einem Reaktor wurden 15,04 kg R113 vorgelegt und auf etwa 75 °C erhitzt. Es wurden 100 g des genannten, herstellungsbedingt Eisentrichlorid enthaltenden AlCl₃ zugesetzt, worauf eine stark exotherme Reation einsetzt (Erwärmung bis auf 116,6 °C). Nach Beendigung der exothermen Reaktion wurde die Reaktionsmischung ca 40 Minuten bei einer Temperatur von 100 °C gerührt. Nach Ziehen einer Probe zwecks Erstellung einer GC-Analyse wurde die Heizung abgeschaltet und das Reaktionsgemisch noch 4 Stunden lang weitergerührt. 2 Stunden und 4 Stunden nach Abschalten der Heizung wurden weitere Proben gezogen.

Die Analysenergebnisse sind im Folgenden zusammengestellt:

GC-Analyse der nach 1 h gezogenen Probe (Angaben in Flächen-%):

| | |
|---|---|
| Gehalt an Verbindungen der Summenformel C₂Cl₃F₃ | 89,02 |
| Gehalt an Verbindungen der Summenformel C₂Cl₂F₄ | 2,95 |
| Gehalt an Verbindungen der Summenformel C₂Cl₄F₂ | 5,94 |
| Gehalt an Verbindungen der Summenformel C₂Cl₆ | 1,21 |
| Gehalt an Verbindungen der Summenformel C₂Cl₅F | 0,83 |

Isomerenverteilung der Verbindungen der Formel C₂Cl₃F₃:

| | |
|---|---|
| R113a | 99,53 |
| R113 | 0,47 |

GC-Analyse der nach 3 h gezogenen Probe (Angaben in Flächen-%):

| | |
|---|---|
| Gehalt an Verbindungen der Summenformel C₂Cl₃F₃ | 89,04 |
| Gehalt an Verbindungen der Summenformel C₂Cl₂F₄ | 2,31 |
| Gehalt an Verbindungen der Summenformel C₂Cl₄F₂ | 4,14 |
| Gehalt an Verbindungen der Summenformel C₂Cl₆ | 3,23 |
| Gehalt an Verbindungen der Summenformel C₂Cl₅F | 1,26 |

Isomerenverteilung der Verbindungen der Formel C₂Cl₃F₃:

| | |
|---|---|
| R113a | 99,73 |
| R113 | 0,27 |

GC-Analyse der nach 5 h gezogenen Probe (Angaben in Flächen-%):

| | |
|---|---|
| Gehalt an Verbindungen der Summenformel C₂Cl₃F₃ | 87,77 |
| Gehalt an Verbindungen der Summenformel C₂Cl₂F₄ | 2,8 |
| Gehalt an Verbindungen der Summenformel C₂Cl₄F₂ | 4,21 |
| Gehalt an Verbindungen der Summenformel C₂Cl₆ | 3,46 |
| Gehalt an Verbindungen der Summenformel C₂Cl₅F | 1,37 |

Isomerenverteilung der Verbindungen der Formel C₂Cl₃F₃:

| | |
|---|---|
| R113a | 99,75 |
| R113 | 0,25 |

Ergebnis: Bereits nach 1 h wurde das eingesetzte R113 annähernd vollständig zum Isomeren R113a umgesetzt, bei lediglich geringfügiger Bildung von Nebenprodukten.

### Beispiel 4:

### Herstellung und Anwendung eines mit AgCl aktivierten Aluminiumtrichlorids.

In einem 300 cm³-Autoklaven aus Edelstahl, der mit Temperaturfühlern für die Messung der Innentemperatur sowie mit einem Manometer ausgestattet war, wurden 300 g eines 113/113a-Gemisches (Gewichtsverhältnis 10,3 : 89,7) mit 33 g eines Umwandlungskatalysators, hergestellt durch Vermischen von 30 g Aluminiumtrichlorid-Pulver (Lieferant: Riedel de Haen) und 3 g AgCl-Pulver, versetzt. Unter Rühren erfolgte binnen 15 Minuten eine Erwärmung auf 91 °C. Das nach Beendigung der Reaktion erhaltene Produkt bestand gemäß gaschromatographischer Analyse zu 87,3 Flächen-% aus 113a (Isomerenreinheit: 100 % !) und einer Gesamtsumme von 12,7 Flächen-% an Nebenkomponenten (114a, 112a, 111 und 110).

Wie diesem Beispiel entnommen werden kann, ist das mit Silberchlorid aktivierte Aluminiumtrichlorid besonders vorteilhaft als Umlagerungskatalysator.

## Patentansprüche

1. Verfahren zur Herstellung von Verbindungen der Formel (I) CF₃CCl₂X mit X = Cl, H, durch Umlagerung von CF₂ClCFCl₂, falls X = Cl ist, oder durch Umlagerung von CF₂ClCHClF oder CF₂HCCl₂F, falls X = H ist, über AlCl₃ als Katalysator, dadurch gekennzeichnet, daß man als Katalysator AlCl₃ einsetzt, welches mittels eines Metallhalogenids ausgewählt aus der Gruppe umfassend AgCl, FeCl₃ und saure Salze der Formel (II) NaF·nHF mit O < n ≤ 2 aktiviert worden ist.

2. Verfahren nach Anspruch 1, dadurch gekennzeichnet, daß man CF₃CCl₃ aus CF₂ClCFCl₂ herstellt.

3. Verfahren nach Anspruch 1 oder 2, dadurch gekennzeichnet, daß man die umzulagernde Ausgangsverbindung CF₂ClCFCl₂ in Form eines Gemisches aus CF₂ClCFCl₂ und CF₃CCl₃ einsetzt.

4. Verfahren nach einem der vorhergehenden Ansprüche, dadurch gekennzeichnet, daß im Salz der Formel (II) 0,5 < n ≤ 2 gilt.

5. Verfahren nach einem der vorhergehenden Ansprüche, dadurch gekennzeichnet, daß die Umlagerung bei einer Temperatur zwischen 70 und 95 °C durchgeführt wird.

6. Verfahren nach einem der vorhergehenden Ansprüche, dadurch gekennzeichnet, daµ AlCl₃ und das Metallhalogenid in einem Gewichtsverhältnis zwischen 5:1 und 100:1 eingesetzt wird.

7. Verfahren nach einem der vorhergehenden Ansprüche, dadurch gekennzeichnet, daß der aktivierte Katalysator in einer Menge von 0,1 bis 10 Gew.-% im Reaktionsgemisch enthalten ist.

8. Verfahren nach einem der vorhergehenden Ansprüche, dadurch gekennzeichnet, daß man AlCl₃ einsetzt, das durch Kontaktieren von AlCl₃ und NaF·HF in CF₂ClCCl₂F erhalten wurde.

9. Verfahren nach Anspruch 8, dadurch gekennzeichnet, daß das Kontaktieren von AlCl₃ und NaF·HF in CF₂ClCCl₂F während einer Zeitdauer von mindestens 1 Stunde durchgeführt wurde.

10. Aluminiumchlorid-Umlagerungskatalysator, erhalten durch Kontaktieren von AlCl₃ und einem Metallhalogenid ausgewählt aus der Gruppe umfassend AgCl, FeCl₃ und Salzen der Formel (II) NaF·nHF mit O < n ≤ 2, mit der Maßgabe, daß im Falle des Kontaktierens mit FeCl₃ der Gehalt an Fe in Form von FeCl₃ mindestens 40 ppm beträgt.

11. Umlagerungskatalysator nach Anspruch 10, erhalten durch Kontaktieren von AlCl₃ und einem Salz der Formel NaF·nHF mit 0 < n ≤ 2 in CF₂ClCCl₂F oder durch Kontaktieren von AlCl₃ mit AgCl.

12. Umlagerungskatalysator nach Anspruch 11, erhalten durch Kontaktieren von AlCl₃ und NaF·HF in CF₂ClCCl₂F über eine Zeitdauer von mindestens 1 Stunde.

13. Aluminiumchlorid-Umlagerungskatalysator, gekennzeichnet durch einen Gehalt an einem Metallhalogenid ausgewählt aus der Gruppe umfassend AgCl, FeCl₃ und sauren Salzen der Formel (II) NaF·nHF mit O < n ≤ 2 mit einer Konzentration an Fe, Ag bzw. Na im Bereich von mehr als 40 bis 10.000 ppm, vorzugsweise 70 bis 150 ppm, in Form von FeCl₃, AgCl oder NaF·nHF, mit der Maßgabe, daß Aluminiumchlorid mit einem Gehalt von bis zu 40 ppm FeCl₃ ausgeschlossen ist.

14. Katalysatorvormischung zur Herstellung des Katalysators gemäß einem der Ansprüche 10 bis 13, bestehend aus 0,1 bis 200 Gewichtsteilen pulverförmigem AlCl₃, 0,01 bis 1 Gewichtsteilen eines Metallhalogenids aus der Gruppe bestehend aus AgCl, FeCl₃ und sauren Salzen der Formel (II) NaF·nHF mit 0 < n ≤ 2 und 0 bis 99,89 Gewichtsteilen CF₂ClCCl₂F.

## Claims

1. Process for preparing compounds of Formula (I) CF₃CCl₂X with X = Cl, H, by rearrangement of CF₂ClCFCl₂, if X = Cl, or by rearrangement of CF₂ClCHClF or CF₂HCCl₂F, if X = H, over AlCl₃ as the catalyst, characterized in that the catalyst employed is AlCl₃ which has been activated by means of a metal halide selected from the group comprising AgCl, FeCl₃ and acid salts of Formula (II) NaF·nHF with 0 < n ≤ 2.

2. Process according to Claim 1, characterized in that CF₃CCl₃ is prepared from CF₂ClCFCl₂.

3. Process according to Claim 1 or 2, characterized in that the initial compound to be rearranged, CF₂ClCFCl₂, is employed in the form of a mixture of CF₂ClCFCl₂ and CF₃CCl₃.

4. Process according to one of the preceding claims, characterized in that for the salt of Formula (II) the relationship 0.5 < n ≤ 2 holds good.

5. Process according to one of the preceding claims, characterized in that the rearrangement is carried out at a temperature between 70 and 95°C.

6. Process according to one of the preceding claims, characterized in that AlCl₃ and the metal halide are used in a weight ratio between 5:1 and 100:1.

7. Process according to one of the preceding claims, characterized in that the activated catalyst is contained in the reaction mixture in an amount of from 0.1 to 10% by weight.

8. Process according to one of the preceding claims, characterized in that AlCl₃ is employed which has been obtained by bringing into contact AlCl₃ and NaF·HF in CF₂ClCCl₂F.

9. Process according to Claim 8, characterized in that the bringing into contact of AlCl₃ and NaF·HF in CF₂ClCCl₂F was carried out for a period of at least 1 hour.

10. Aluminium chloride rearrangement catalyst, obtained by bringing into contact AlCl₃ and a metal halide, selected from the group comprising AgCl, FeCl₃ and salts of Formula (II) NaF·nHF with 0 < n ≤ 2, with the proviso that, in the case of bringing into contact with FeCl₃, the content of Fe in the form of FeCl₃ is at least 40 ppm.

11. Rearrangement catalyst according to Claim 10, obtained by bringing into contact AlCl₃ and a salt of Formula NaF·nHF with 0 < n ≤ 2 in CF₂ClCCl₂F or by bringing into contact AlCl₃ with AgCl.

12. Rearrangement catalyst according to Claim 11, obtained by bringing into contact AlCl₃ and NaF·HF in CF₂ClCCl₂F for a duration of at least 1 hour.

13. Aluminium chloride rearrangement catalyst, characterized by a content of a metal halide selected from the group comprising AgCl, FeCl₃ and acid salts of Formula (II) NaF·nHF with 0 < n ≤ 2, with a concentration of Fe, Ag and Na in the range of from more than 40 to 10,000 ppm, preferably from 70 to 150 ppm, in the form of FeCl₃, AgCl or NaF·nHF, with the proviso that aluminium chloride with a content of up to 40 ppm of FeCl₃ is excluded.

14. A catalyst premix for preparing the catalyst according to one of Claims 10 to 13, consisting of 0.1 to 200 parts by weight powdered AlCl₃, 0.01 to 1 parts by weight of a metal halide from the group consisting of AgCl, FeCl₃ and acidic salts of Formula (II) NaF·nHF with 0 < n ≤ 2 and 0 to 99.89 parts by weight CF₂ClCCl₂F.

## Revendications

1. Procédé de préparation de composés de formule (I) CF₃CCl₂X, dans laquelle X représente Cl, H, par transposition de CF₂ClCFCl₂, si X représente Cl ou par transposition de CF₂ClCHClF ou CF₂HCCl₂F, si X représente H, sur AlCl₃ comme catalyseur, procédé caractérisé en ce qu'on utilise comme catalyseur AlCl₃ qui a été activé à l'aide d'un halogénure de métal choisi dans le groupe ou ensemble comprenant AgCl, FeCl₃, et des sels acides de formule (II) NaF.nHF, dans laquelle 0 < n ≤ 2.

2. Procédé selon la revendication 1, caractérisé en ce qu'on produit CF₃CCl₃ à partir de CF₂ClCFCl₂.

3. Procédé selon la revendication 1 ou 2, caractérisé en ce qu'on utilise le composé de départ CF₂ClCFCl₂ à transposer sous la forme d'un mélange de CF₂ClCFCl₂ et de CF₃CCl₃.

4. Procédé selon l'une des revendications précédentes, caractérisé en ce que, dans le sel de formule (II), on a 0,5 < n ≤ 2.

5. Procédé selon l'une des revendications précédentes, caractérisé en ce que la transposition est effectuée à une température comprise entre 70 et 95°C.

6. Procédé selon l'une des revendications précédentes, caractérisé en ce qu'on utilise AlCl₃ et l'halogénure de métal selon un rapport pondéral compris entre 5:1 et 100:1.

7. Procédé selon l'une des revendications précédentes, caractérisé en ce que le catalyseur activé est contenu en une quantité de 0,1 à 10% en poids dans le mélange réactionnel.

8. Procédé selon l'une des revendications précédentes, caractérisé en ce qu'on utilise AlCl₃ qui a été obtenu par mise en contact de AlCl₃ et de NaF.HF dans CF₂ClCCl₂F.

9. Procédé selon la revendication 8 caractérisé en ce qu'on a effectué la mise en contact de AlCl₃ et de NaF.HF dans CF₂ClCCl₂F durant une période de temps d'au moins une heure.

10. Catalyseur de transposition à base de chlorure d'aluminium, obtenu par mise en contact de AlCl₃ et d'un halogénure de métal choisi dans l'ensemble comprenant AgCl, FeCl₃ et des sels de formule (II) NaF.nHF avec 0 < n ≤ 2, à la condition que, dans le cas de la mise en contact avec FeCl₃, la teneur en Fe sous forme de FeCl₃ vale au moins 40 ppm.

11. Catalyseur de transposition selon la revendication 10, obtenu par mise en contact de AlCl₃ et d'un sel de formule NaF.nHF avec 0 < n ≤ 2 dans CF₂ClCCl₂F ou par mise en contact de AlCl₃ avec AgCl.

12. Catalyseur de transposition selon la revendication 11, obtenu par mise en contact de AlCl₃ et de NaF.HF dans CF₂ClCCl₂F pendant un intervalle de temps d'au moins une heure.

13. Catalyseur de transposition à base de chlorure d'aluminium, caractérisé par une teneur en un halogénure de métal choisi dans l'ensemble comprenant AgCl, FeCl₃ et des sels acides de formule (II) NaF.nHF, avec 0 < n ≤ 2, avec une concentration en Fe, Ag ou Na se situant dans la gamme de plus de 40 ppm à 10 000 ppm, avantageusement 70 à 150 ppm, sous forme de FeCl₃, AgCl ou NaF.nHF, à la condition que soit exclu du chlorure d'aluminium ayant une teneur en FeCl₃ allant jusqu'à 40 ppm de FeCl₃.

14. Prémélange de catalyseur pour la préparation du catalyseur selon l'une des revendications 10 à 13, consistant en 0,1 à 200 parties en poids de AlCl₃ pulvérulent, 0,01 à 1 partie en poids d'un halogénure de métal choisi dans l'ensemble consistant en AgCl, FeCl₃ et des sels acides de formule (II) NaF.nHF avec 0< n ≤ 2, et de 0 à 99,89% parties en poids de CF₂ClCCl₂F.
